# EUROPEAN PATENT APPLICATION

(11) **EP 4 559 453 A1**
(43) Date of publication of application: **28.05.2025**
(21) Application number: 24214816.1
(22) Date of filing: 22.11.2024
(51) Int. Cl.: A61K 8/98, A61K 9/00, A61K 35/20, A61P 17/10, A61Q 3/00, A61Q 5/02, A61Q 5/12, A61Q 19/00, A61Q 19/08, A61Q 19/10

(54) **COSMETIC COMPOSITION COMPRISING OVINE COLOSTRUM, METHOD OF PRODUCING THEREOF AND USE THEREOF**

(30) Priority: 22.11.2023 PL 44680123
(71) Applicant: Uniwersytet Medyczny w Lodzi, 90-419 Lodz (PL)
(72) Inventor: Kalinowska-Lis, Urszula, Lódz (PL); Kazimierska, Kinga, Warszawa (PL)
(74) Representative: Wlasienko, Jozef

(57) **Abstract**

The subject matter of the invention is a cosmetic composition comprising:
- 0.1% to 50% by weight of ovine colostrum being the active substance; and
- a cosmetic base comprising at least one preservative, at least one emulsifier and at least one solvent.

The subject matter of the invention is also a method of producing the above-described composition and its use for improving skin condition and in the topical treatment of acne.

## Description

The subject matter of the invention is a cosmetic composition, a method of producing the composition and its use for improving skin condition, especially skin with aging signs and acne skin.

Presently, several types of colostrum are used on the market depending on its origin (usually cow, bovine and caprine). Colostrum is usually found in dietary supplements (mainly caprine) but also in cosmetics (mainly bovine).

According to the data included in the review article by K. Kazimierska et al.: "Milk Proteins-Their Biological Activities and Use in Cosmetics and Dermatology", Molecules 2021, 26, 3253 cosmetic compositions containing bovine or equine colostrum promote the healing of burn wounds, ulcers and sunburns, and are effective in the care of skin with psoriatic lesions (they reduce skin redness and roughness). Furthermore, creams based on bovine and/or equine milk and/or colostrum improve the condition of skin with acne rosacea, atopic dermatitis and common acne. One study also showed the anti-aging efficacy of a cream with bovine and donkey colostrum. However, most of these studies relate to the synergistic effect of milk-derived ingredients and other active and auxiliary substances, such as vitamin E, zinc, arbutin, hyaluronic acid, squalene, zinc oxide, panthenol, which does not allow one to draw unambiguous conclusions on the independent effect of colostrum in preparations.

Patent document CA2450558A1 discloses a cosmetic composition based on ovine milk - not ovine colostrum - and a method of preparing the composition. According to the disclosure the composition can be used for skin, nails and hair.

Patent document CN102309430A discloses the use of fat isolated from ovine colostrum for the production of facial masks.

Ovine colostrum as a raw material has a rich protein profile, equally rich profile of fatty acids, including polyunsaturated fatty acids, and contains vitamins and mineral salts. Ovine colostrum contains about twice as much proteins and fats as bovine or caprine colostrum, which is its clear advantage over colostrum of other origin. Moreover, ovine colostrum is characterized by a very high content of immunoglobulins and growth factors. The content of growth factors (especially EGF) is of great importance in the context of the influence of colostrum on skin. The content of EGF in ovine colostrum is about ten times higher than in bovine colostrum. Despite such advantageous properties of ovine colostrum, cosmetic compositions comprising ovine colostrum in their content are not known in the state of the art.

Therefore, the purpose of the present invention is to provide a cosmetic composition comprising ovine colostrum for external use on healthy or affected skin, which does not cause adverse reactions, e.g. allergic reactions.

The subject matter of the invention is a cosmetic composition comprising:
- 0.1% to 50% by weight of ovine colostrum being the active substance; and
- a cosmetic base comprising at least one preservative, at least one emulsifier and at least one solvent.

Preferably, the composition according to the invention comprises the at least one preservative in a total amount of 0.001% to 2.5% by weight relative to the total mass of the composition.

Preferably, the composition according to the invention comprises the at least one emulsifier in a total amount of 1% to 25% by weight relative to the total mass of the composition.

Preferably, the composition according to the invention comprises the at least one solvent in an amount that is a complement to 100% by weight relative to the total mass of the composition.

Preferably, the cosmetic base additionally comprises at least one ingredient selected from the group consisting of at least one: thickener, humectant, emollient, antioxidant, fragrance, pH regulator, surfactant, ultraviolet filter, silicone, extender and colourant.

Preferably, the composition according to the invention comprises the at least one thickener in a total amount of 1% to 60% by weight relative to the total mass of the composition.

Preferably, the composition according to the invention comprises the at least one humectant in a total amount of 1% to 50% by weight relative to the total mass of the composition.

Preferably, the composition according to the invention comprises the at least one emollient in a total amount of 1% to 25% by weight relative to the total mass of the composition.

Preferably, the composition according to the invention comprises the at least one antioxidant in a total amount of 0.01% to 10% by weight relative to the total mass of the composition.

Preferably, the composition according to the invention comprises the at least one fragrance in a total amount of 0.01% to 3% by weight relative to the total mass of the composition.

Preferably, the composition according to the invention comprises the at least one pH regulator in a total amount sufficient to obtain a pH value in the range of 4.5 to 6.0.

Preferably, the composition according to the invention comprises the at least one surfactant selected from the group consisting of anionic, cationic, amphoteric and non-ionic surfactants in a total amount of 0.1% to 5% by weight relative to the total mass of the composition.

Preferably, the composition according to the invention comprises the at least one ultraviolet filter in a total amount of 1% to 30% by weight relative to the total mass of the composition.

Preferably, the composition according to the invention comprises the at least one silicone in a total amount of 1% to 50% by weight relative to the total mass of the composition.

Preferably, the composition according to the invention comprises the at least one extender in a total amount of 0.1% to 10% by weight relative to the total mass of the composition.

Preferably, the composition according to the invention comprises the at least one colourant in a total amount of 0.1% to 5% by weight relative to the total mass of the composition.

Preferably, ovine colostrum is in the form of lyophilizate, in liquid form or in partially concentrated form.

Preferably, the composition according to the invention is in the form of: cream, ointment, serum, facial and body mask, exfoliator, balm, gel, shampoo, hair mask, hair conditioner, lip care stick or nail conditioner.

The subject matter of the invention is also a method of producing the composition as defined above, wherein the method comprises the following steps:
a) introducing a solvent forming phase I into a mixer and heating it to a temperature falling within the range of 20 to 80°C;
b) introducing an emulsifier forming phase II into a melting crucible and then heating it to a temperature falling within the range of 20 to 80°C and mixing it to obtain a homogenous phase;
c) dosing phase II from step b) to phase I from step a) and simultaneously mixing to obtain phase III in a homogenous form;
d) adding, to phase III obtained in step c), successively, ovine colostrum and a preservative to obtain the composition; wherein each time after adding a successive ingredient, phase III is thoroughly mixed until it is homogenous.

Preferably, the method of producing according to the invention further comprises, after step d), the step of:
e) cooling down the composition obtained in step d) to room temperature.

Preferably, the method of producing according to the invention further comprises, after step d) or e), the step of:
f) adding a pH regulator in an amount sufficient to obtain a pH value in the range of 4.5 to 6.0 to the composition obtained in step d) or e), wherein the adding is carried out in portions and with mixing.

Preferably, the method of producing according to the invention further comprises, between steps c) and d), the step of:
c') cooling down phase III obtained in step c) to a temperature below 40°C.

Preferably, in step a), at least one additional ingredient forming phase I, selected from the group consisting of at least one: thickener, humectant and surfactant, is introduced into the mixer while mixing.

Preferably, in step b), at least one additional ingredient forming phase II, selected from the group consisting of at least one: antioxidant and emollient, is introduced into the melting crucible.

Preferably, in step d), at least one additional ingredient selected from the group consisting of at least one: fragrance, colourant, antioxidant, ultraviolet filter, silicone, extender and humectant is added.

Preferably, the at least one emulsifier is introduced in step b) in such an amount that the total amount of the at least one emulsifier in the composition is 1% to 25% by weight relative to the total mass of the composition.

Preferably, ovine colostrum is added in step d) in such an amount that its amount in the composition is 0.1% to 50% by weight relative to the total mass of the composition.

Preferably, the at least one preservative is added in step d) in such an amount that the total amount of the at least one preservative in the composition is 0.001% to 2.5% by weight relative to the total mass of the composition.

Preferably, the at least one thickener is introduced in step a) in such an amount that the total amount of the at least one thickener in the composition is 1% to 60% by weight relative to the total mass of the composition.

Preferably, the at least one humectant is introduced in step a) and/or in step d) in such an amount that the total amount of the at least one humectant in the composition is 1% to 50% by weight relative to the total mass of the composition.

Preferably, the at least one surfactant is introduced in step a) in such an amount that the total amount of the at least one surfactant in the composition is 0.1% to 5% by weight relative to the total mass of the composition.

Preferably, the at least one antioxidant is introduced in step b) and/or in step d) in such an amount that the total amount of the at least one antioxidant in the composition is 0.01% to 10% by weight relative to the total mass of the composition.

Preferably, the at least one emollient is introduced in step b) in such an amount that the total amount of the at least one emollient in the composition is 1% to 25% by weight relative to the total mass of the composition.

Preferably, the at least one fragrance is added in step d) in such an amount that the total amount of the at least one fragrance in the composition is 0.01% to 3% by weight relative to the total mass of the composition.

Preferably, the at least one colourant is added in step d) in such an amount that the total amount of the at least one colourant in the composition is 0.1% to 5% by weight relative to the total mass of the composition.

Preferably, the at least one ultraviolet filter is added in step d) in such an amount that the total amount of the at least one ultraviolet filter in the composition is 1% to 30% by weight relative to the total mass of the composition.

Preferably, the at least one silicone is added in step d) in such an amount that the total amount of the at least one silicone in the composition is 1% to 50% by weight relative to the total mass of the composition.

Preferably, the at least one extender is added in step d) in such an amount that the total amount of the at least one extender in the composition is 0.1% to 10% by weight relative to the total mass of the composition.

Preferably, the at least one solvent is introduced in step a) in such an amount that the total amount of the at least one solvent in the composition is a complement to 100% by weight relative to the total mass of the composition.

Moreover, the subject matter of the invention is a use of the composition as defined above for improving skin condition.

Preferably, improving skin condition comprises: increasing skin hydration, increasing skin elasticity, reducing redness, regenerating aging skin, reducing skin hypersensitivity, evening out skin tone and smoothing skin pores.

The subject matter of the invention is the composition as defined above for use in the topical treatment of acne.

The solutions according to the invention make it possible to obtain a composition which comprises ingredients derived from natural sources and the use of which is associated with a low risk of adverse reactions, e.g. allergic reactions. The advantage of using ovine colostrum as the active substance in a cosmetic composition over other active substances is that a multifaceted beneficial effect on skin is obtained using only one multicomponent raw material in "natural" proportions. The cosmetic composition according to the invention comprising ovine colostrum has a number of advantageous effects on healthy skin, such as increasing skin hydration, increasing skin elasticity, reducing redness, regenerating aging skin, reducing skin hypersensitivity, evening out skin tone and smoothing skin pores, as well as protective, firming and sebostatic effects.

These and other advantages of the present invention will be obvious in the light of the following description of the invention, in particular its embodiments.

As used in the present description, the terms "include" and "comprise" are not limiting. The terms "include" and "comprise" should be interpreted to mean that the indicated features are included but the presence of other features is not excluded.

Below is presented a detailed description of the invention.

Ovine colostrum is defined as the secretion of the mammary gland of sheep produced in the last period before giving birth and in the first days (max. three days) after giving birth. Colostrum taken within 2 hours after giving birth has the highest amount of bioactive ingredients. It is best to take the raw material no later than 1 day after giving birth. The raw material taken into sterile vessels, pure and unprocessed in any way, should be frozen immediately after milking, except when immediately used.

Ovine colostrum is a completely safe, 100% natural raw material. This raw material can be used in the form of lyophilizate (powder), in liquid form or in partially concentrated form, preferably in the form of lyophilizate. The gentle process of lyophilization - freeze drying under reduced pressure, makes it possible to preserve the properties of bioactive ingredients of colostrum. It is recommended not to subject the raw material to pasteurization; however, the use of pasteurized (low-temperature pasteurization) or microfiltered raw material is not excluded. The raw material in a selected form should be tested for microbiological purity before being included in a composition. The raw material can be subjected to gamma radiation in order to eliminate pathogenic microorganisms, if any.

According to the invention, colostrum as the active substance is used in combination with cosmetic base ingredients including a solvent, emulsifier and preservative; wherein more than one type of solvent, emulsifier and preservative can be used.

Additional cosmetic base ingredients selected from the group consisting of a thickener, humectant, emollient, antioxidant, fragrance, pH regulator, surfactant, ultraviolet filter, silicone, extender and colourant can be added to the composition comprising colostrum. More than one type of given ingredient (for example, two different thickeners) or any combination of the above specified ingredients can be used.

A suitable preservative for use in the solutions according to the present invention is potassium sorbate, methylparaben, sodium benzoate, phenoxyethanol or triclosan.

At least one preservative is included in the composition in a total amount of 0.001% to 2.5% by weight relative to the total mass of the composition.

A suitable emulsifier for use in the solutions according to the present invention is glyceryl oleate, glyceryl stearate, glyceryl stearate citrate, lanoline, lecithin, potassium stearate, sorbitan laurate, stearic acid, sucrose myristate, sucrose laurate, 1,2,3-propanetriol monooctadecanoate, cetearyl olivate or sorbitan olivate.

At least one emulsifier is included in the composition in a total amount of 1% to 25% by weight relative to the total mass of the composition.

A suitable solvent for use in the solutions according to the present invention is distilled water, spring water, thermal water, hydrolat, ethanol, glycerine or vegetable oil.

At least one solvent is included in the composition in an amount that is a complement to 100% by weight relative to the total mass of the composition.

A suitable thickener for use in the solutions according to the present invention is acrylate copolymer, sodium salt of alginic acid, carboxymethyl cellulose, hydroxypropyl cellulose, hydroxyethyl cellulose, carrageenan or carrageenan sodium salt.

At least one thickener is included in the composition in a total amount of 1% to 60% by weight relative to the total mass of the composition.

A suitable humectant for use in the solutions according to the present invention is glycerine, sorbitol, pentylene glycol, urea, sodium lactate or amino acid such as histidine.

At least one humectant is included in the composition in a total amount of 1% to 50% by weight relative to the total mass of the composition.

A suitable emollient for use in the solutions according to the present invention is isononyl isononanoate, cetyl alcohol, tripelargonin, shea butter or avocado oil.

At least one emollient is included in the composition in a total amount of 1% to 25% by weight relative to the total mass of the composition.

A suitable antioxidant for use in the solutions according to the present invention is alpha-tocopherol, sodium ascorbyl phosphate, butylhydroxytoluene or *Helianthus Annuus* seed oil.

At least one antioxidant is included in the composition in a total amount of 0.01% to 10% by weight relative to the total mass of the composition.

A suitable fragrance for use in the solutions according to the present invention is anise alcohol, eucalyptole, *Jasminum officinale, Lavandula officinalis,* menthol, 2-octyldodecan-1-ol, lemon, linalool, vanillin or parfum.

At least one fragrance is included in the composition in a total amount of 0.01% to 3% by weight relative to the total mass of the composition.

A suitable pH regulator for use in the solutions according to the invention is citric acid, lactobionic acid, sodium lactate or ammonium carbonate.

At least one pH regulator is included in the composition in a total amount sufficient to obtain a pH value in the range of 4.5 to 6.0.

A suitable surfactant for use in the solutions according to the present invention is both anionic, cationic, amphoteric and non-ionic surfactant, such as caprylyl glucoside, capryl glucoside, cocamidopropyl betaine or ethoxylated sodium lauryl sulphate.

At least one surfactant is included in the composition in a total amount of 0.1% to 5% by weight relative to the total mass of the composition.

A suitable ultraviolet filter for use in the solutions according to the present invention is benzophenone-3, zinc oxide, octocrylene or bis-ethylhexyloxyphenol methoxyphenyl triazine.

At least one ultraviolet filter is included in the composition in a total amount of 1% to 30% by weight relative to the total mass of the composition.

A suitable silicone for use in the solutions according to the present invention is cyclopentasiloxane, dimethicone, amodimethicone or silane such as alkoxysilane.

At least one silicone is included in the composition in a total amount of 1% to 50% by weight relative to the total mass of the composition.

A suitable extender for use in the solutions according to the present invention is starch, kaolin, nylon powder or talc.

At least one extender is included in the composition in a total amount of 0.1% to 10% by weight relative to the total mass of the composition.

A suitable colourant for use in the solutions according to the present invention is zinc stearate, capsanthin, disodium 6-hydroxy-5-[(4-sulphonatophenyl)azo]naphthalene-2-sulphonate, titanium dioxide or magnesium carbonate.

At least one colourant is included in the composition in a total amount of 0.1% to 5% by weight relative to the total mass of the composition.

When producing the cosmetic composition according to the invention, the ingredients of the composition referred to above are introduced into phase I or phase II, or phase III obtained after combining these phases, or are only added to the cosmetic composition, depending on their properties (hydrophilic or lipophilic) and sensitivity to temperature.

First, a solvent is introduced into a mixer, which is then heated to a temperature in the range of 20 to 80°C (step a). The solvent forms phase I.

Separately, an emulsifier is introduced into a melting crucible, which is also heated to a temperature in the range of 20 to 80°C (step b). Thus, the emulsifier forms phase II.

After melting the emulsifier and mixing it in order to obtain a homogenous form, it is dosed into the solvent and the whole is mixed to obtain phase III in a homogenous form (step c).

In the case the above steps are carried out at a temperature above 40°C, the obtained phase III should be cooled down to a temperature below 40°C (step c') before the next step (step d), in which ingredients sensitive to temperature are sequentially added, i.e. ovine colostrum as the active substance and a preservative. In step d) individual ingredients are added sequentially. After adding each of the ingredients, phase III is mixed to obtain a homogenous form, before adding a successive ingredient. As a result of this step (step d) the cosmetic composition is obtained.

In some embodiments, when the temperature of the above-mentioned cosmetic composition is higher than room temperature, the composition is cooled down to room temperature (step e).

In the final step (step f) a pH regulator can be added to the cosmetic composition in an amount sufficient to obtain a pH of the composition in the range of 4.5 to 6.0, the adding being carried out in portions and with mixing. The pH regulator can be added to the composition obtained in step d). In some embodiments, when the composition obtained in step d) is cooled down to room temperature, the pH regulator is added to the composition only after it has been cooled down (after step e).

In step a), at least one additional ingredient selected from the group consisting of at least one thickener, at least one humectant and at least one surfactant can be introduced into phase I; preferably at least one thickener and at least one humectant is introduced.

In step b), at least one additional ingredient selected from the group consisting of at least one antioxidant and at least one emollient can be introduced into phase II.

In step d), at least one additional ingredient selected from the group consisting of at least one fragrance, at least one colourant, at least one antioxidant, at least one ultraviolet filter, at least one silicone, at least one extender and at least one humectant can be added to phase III obtained by combining phase I and phase II; preferably a fragrance and a humectant are added.

As indicated above, an antioxidant can be introduced into either phase II (in step b) or phase III (in step d) or into both of these phases, depending on the antioxidant used and its properties; for example, if it is sensitive to temperature, it is introduced only to phase III, obtained by combining phase I and phase II, which has a temperature lower than 40°C.

Furthermore, a humectant can be introduced into either phase I (in step a) or phase III (in step d) or into both of these phases, depending on the humectant used and its properties and on the content of the composition and its properties.

All the above-mentioned ingredients are introduced in such amounts that their contents in the final composition correspond to the values indicated above in the description of the composition.

The cosmetic composition according to the invention is used in the form of preparations such as cream, emulsion, ointment, gel, mask, balm, oil, stick, shampoo, hair conditioner, etc.

The cosmetic composition according to the invention, due to its properties, is used for improving skin condition, in particular for increasing skin hydration and elasticity, reducing redness, regenerating aging skin, reducing skin hypersensitivity, evening out skin tone and smoothing skin pores. Additionally, it is also used for the local treatment of acne.

The invention is illustrated by means of the following example, which however does not limit it.

In the first step, phase I was produced in the following way: acrylate/C₁₀₋₃₀ alkyl acrylate crosspolymer (INCI: Acrylates/C10-30 Alkyl Acrylate Crosspolymer) was added dropwise to water and left until its complete wetting, and mixing was started; then it was heated to the temperature of 70-75°C and pentylene glycol was introduced.

Then, in the second step, phase II was produced by introducing isononyl isononanoate, cetearyl olivate, sorbitan olivate, glyceryl stearate citrate, tripelargonin, *Helianthus Annuus* seed oil and tocopherol into a melting crucible. The whole was heated to the temperature of 75°C and was mixed to obtain homogenous phase II.

In the next step, phase II was combined with phase I and homogenized under vacuum to produce phase III.

In the next step, after cooling down phase III to a temperature below 40°C, temperature-sensitive ingredients of phase III were sequentially added, including ovine colostrum, aqueous solution of sodium lactate, aqueous solution of potassium sorbate and sodium benzoate, parfum; wherein after adding each ingredient, the mixture was thoroughly mixed to obtain a homogenous composition.

After adding all ingredients, the obtained composition was at room temperature.

In the last step, a solution of citric acid was added (phase IV) in order to adjust the pH of the composition to the range of 4.7-5.2. Table 1 shows the ingredients of the cosmetic composition according to the invention and their contents in % by weight relative to the total mass of the composition.

**Table 1. The cosmetic composition according to the invention.**

| Phase | INCI name | Function | Content [% by weight.] |
|---|---|---|---|
| I | Water | Solvent | Complement to 100.0 |
| | Acrylates/C10-30 Alkyl Acrylate Crosspolymer | Thickener | 0.2 |
| | Pentylene glycol | Humectant | 3.0 |
| II | Isononyl isononanoate | Emollient | 7.0 |
| | Cetearyl olivate, sorbitan olivate | Emulsifier | 4.5 |
| | Glyceryl stearate citrate | Emulsifier | 2.0 |
| | Tripelargonin | Emollient | 7.0 |
| | *Helianthus Annuus seed oil,* tocopherol | Antioxidant | 0.1 |
| III | Parfum | Fragrance | 0.7 |
| | Ovine colostrum | Active substance | 15.0 |
| | Aqueous solution of sodium lactate | Humectant | 0.5 |
| | Aqueous solution of potassium sorbate and sodium benzoate | Preservative | 1.5 |
| IV | Aqueous solution of citric acid | pH regulator | 1.3-1.5 |

The cosmetic composition produced above was subjected to a series of tests in accordance with the requirements of the Regulation (EC) No 1223/2009 of the European Parliament and of the Council of 30 November 2009 on cosmetic products by an accredited laboratory. The scope of tests included: a test for microbiological purity, a test for the effectiveness of the preservative system used, a test for stability and compatibility with packaging, and a dermatological test (a semi-open test on 25 volunteers). The tests confirmed the safety of the above obtained cosmetic composition.

### Testing the cosmetic composition according to the invention in the form of cream on a group of women having skin with aging signs

Studies were carried out with the approval of the Bioethics Commission.

An eight-week, double-blind, randomized, placebo-controlled study was carried out in order to test the clinical efficacy of the cosmetic composition according to the invention in the care of aging skin. The study involved 48 women aged 40-70 years (the average age in the group was 50.4).

Participants were randomly enrolled to two groups: *the colostrum group* (30 participants) and *the placebo group* (18 participants). The tested cosmetic composition was applied on the facial skin once a day, at night. Both an objective and subjective assessment of the skin of the participants was made. In order to objectively assess the skin condition of the subjects, during the first visit and after 8 weeks of using the cosmetic composition according to the invention, skin parameters on the cheeks were measured using Courhage - Khazaka Multi Probe Adapter (MPA 580), checking the level of hydration and lubricating, elasticity, melanin content, erythema and the degree of transepidermal water loss (TEWL) from the skin. The study also included photodocumentation of the skin of the participants before and after using the cosmetic composition according to the invention as well as the subjects' subjective assessment of the effects of the therapy with the cosmetic composition.

### Results of testing the cosmetic composition according to the invention in the form of cream

### 1. Instrumental studies of skin parameters

Skin hydration: A statistically significant improvement in skin hydration was noted after using the tested cosmetic composition in the *colostrum group,* in which the average value of skin hydration increased from 35.9 to 51.1 units. In contrast, in the *placebo group,* the change in skin hydration was small (from 42.2 to 46.2 units) and statistically insignificant.

TEWL: In both groups, significant improvements in TEWL values were observed after 8 weeks of using the cosmetic composition according to the invention, shifting from the normal level (15-25 g/h/m2) to a healthier condition (10-15 g/h/m2).

Skin elasticity as assessed by the R0 and F0 parameters by means of Cutometer, was improved after using the tested cosmetic composition in both study groups, with R0 representing the passive behaviour of the skin to force, lower values indicating greater firmness; and F0 representing skin firmness at the moment of suction, lower values indicating more elastic skin.

The remaining skin parameters tested, i.e. erythema, melanin content and sebum, did not change significantly in both groups after 8 weeks.

However, it is worth mentioning that 3 subjects from *the colostrum group* noticed that their oily skin reduced sebum secretion after the use of the tested cosmetic composition and, indeed, their observations were consistent with the sebum values recorded for those subjects by the instruments.

### 2. Photodocumentation

The study was a double-blind study, that is, the researchers assessed "blinded" photos without knowing whether the subjects belonged to the *placebo group* or the *colostrum group.* Statistically significant differences between the groups were obtained for two parameters: reduction of redness and smoothing of skin pores, where an improvement was noted by 25.0% and 71.4% of the subjects, respectively, in the *colostrum group,* whereas in the *placebo group,* by 0% and 38.9% of the subjects, respectively. A significant improvement, noted by 71.4% and 53.6% of the subjects from the *colostrum group,* was also obtained in the assessment of "overall improvement" and "evening out of skin tone". The last parameter assessed, i.e. wrinkle shallowing, was also characterized by a greater improvement compared to the *placebo group.*

### 3. Subjective assessment by subjects.

In general, the percentage of the subjects experiencing an improvement in skin condition in the *colostrum group* was from 27.6% to 55.2%, whereas in the *placebo group,* it was from 17.6% to 40.0%. In the *colostrum group,* about 50% of the subjects declared an improvement in skin hydration, regeneration and softness, as well as redness reduction and skin hypersensitivity reduction.

### Summary and conclusions from the studies on the group of women having skin with aging signs

The results indicated a higher efficacy of the cosmetic composition according to the invention in improving skin condition compared to placebo. In summary, it was found that the use of the cosmetic composition according to the invention resulted in significant objective improvements in various aspects of skin appearance, particularly improvements in hydration, TEWL and elasticity, suggesting potential benefits for its future dermatological and aesthetic applications.

The cosmetic composition according to the invention is a universal product; *inter alia* it causes an improvement in the hydrolipid barrier of the skin. It also shows anti-aging effect, not only in the aspect of improvement in skin hydration, TEWL, softness and elasticity, but also in terms of skin tone alignment, regeneration, smoothing of skin pores, redness reduction, which was confirmed by the photos of the skin "before" and "after" the studies and subjective assessments of the subjects.

### Testing the cosmetic composition according to the invention on a group of persons having acne skin

Studies were carried out with the approval of the Bioethics Commission. An eight-week study was carried out to test the efficacy of the cosmetic composition according to the invention in the care of acne skin. The study involved 27 persons aged 18-35 years. It was not a placebo-controlled study.

Participants applied the tested cosmetic composition on their facial skin twice a day, in the morning and at night. Both an objective and subjective assessment of the skin of the participants was made. In the objective assessment - by means of instruments, such parameters as hydration, TEWL and sebum (MPA 580) were considered. The skin on the forehead, chin and cheeks was examined. The study also included the subjects' subjective assessment of the effects of the therapy with the cosmetic composition according to the invention.

### Results of the study:

### 1. Instrumental studies of skin parameters

A statistically significant improvement in skin hydration on the forehead and cheeks, an improvement in the TEWL values on the cheeks and chin and seborrhea reduction on the forehead were noted after using the cosmetic composition according to the invention for 8 weeks. In the remaining facial parts examined, these parameters were also improved but the results did not show statistical significance. Table 2 shows statistical data concerning the instrumental studies of the skin parameters.

**Table 2. Statistical data concerning the instrumental studies of the skin parameters.**

| **N=27** | **Before** | **After** | **Significance** |
|---|---|---|---|
| **Hydration** | | | |
| Forehead | 50.7±13.8 | 56.6±10.0 | P=0.036 |
| | 51.0 (42.2-58.2) | 58.5 (53.2-61.0) | |
| Cheek | 43.2±12.8 | 49.3±11.4 | P=0.013 |
| | 41.0 (38.5-52.0) | 50.0 (41.5-55.7) | |
| Chin | 50.1±10.9 | 54.9±19.7 | NS (P=0.171) |
| | 50.0 (41.5-54.2) | 52.0 (40.7-60.0) | |

| **TEWL** | | | |
|---|---|---|---|
| Forehead | 17.3±9.1 | 16.8±6.1 | NS (P=0.876) |
| | 15.5 (10.0-20.5) | 15.0 (13.0-18.5) | |
| Cheek | 20.1±6.5 | 16.5±6.2 | P=0.013 |
| | 18.5 (14.7-25.0) | 15.0 (13.0-16.2) | |
| Chin | 26.4±11.9 | 18.9±8.4 | P<0.001 |
| | 22.0 (17.7-33.0) | 16.0 (14.0-20.0) | |

| **Sebum** | | | |
|---|---|---|---|
| Forehead | 132.8±67.8 | 91.7±41.0 | P=0.002 |
| | 120.5 (81.5-199.7) | 84.0 (61.0-101.2) | |
| Cheek | 63.3±62.1 | 69.2±52.7 | NS (P=0.264) |
| | 51.5 (22.2-69.2) | 64.0 (35.0-83.7) | |
| Chin | 110.2±65.3 | 103.1±56.2 | NS (P=0.207) |
| | 89.5 (62.7-145.0) | 90.0 (60.5-134.2) | |

| | | | |
|---|---|---|---|
| Values in the Table: Mean ±SD, Median (25-75) (interquartile 25% and 75%) | | | |

### 2. Subjective assessment by the subjects

The percentage of the subjects experiencing an improvement in the reduction of blackheads, papules, pustules and erythema was 68%, 78%, 75% and 82%, respectively.

The average improvement in the group declaring an improvement was 38% (reduction of blackheads), 39% (reduction of papules), 43% (reduction of pustules) and 38% (reduction of erythema). Table 3 shows data concerning the subjects' subjective assessment regarding the reduction of blackheads, papules, pustules and erythema.

**Table 3. Data concerning the subjects' subjective assessment regarding the reduction of blackheads, papules, pustules and erythema.**

| | **BLACKHEADS** | | | **PAPULES** | | | **PUSTULES** | | | **ERYTHEMAS** | | |
|---|---|---|---|---|---|---|---|---|---|---|---|---|
| | **forehead** | **chin** | **cheeks** | **forehead** | **chin** | **cheeks** | **forehead** | **chin** | **cheeks** | **forehead** | **chin** | **cheeks** |
| Average improvement in the group declaring an improvement (size of the group) | 33.3% (N=18) | 38.8% (N=12) | 40.4% (N=13) | **32.6% (N=21)** | **43.2 % (N=16)** | **41.8% (N=17)** | 37.6% (N=21) | 44.3% (N=15) | 48.3% (N=15) | **40.4% (N=16)** | **31.5% (N=10)** | **43.5% (N=12)** |
| Size of the group with no changes | N=3 | N=3 | N=3 | **N=1** | **N=2** | **N=2** | N=1 | N=1 | N=0 | **N=0** | **N=1** | **N=1** |
| Average deterioration in the group declaring deterioration (size of the group) | 12.5% (N=2) | 10.7% (N=7) | 10% (N=2) | **15.0% (N=2)** | **16.0% (N=5)** | **30.0% (N=3)** | 10.0% (N=3) | 19.5% (N=6) | 30.8% (N=6) | **10.0% (N=2)** | **10.0% (N=1)** | **8.3% (N=3)** |
| Size of the group with no symptoms | N=4 | N=5 | N=9 | **N=3** | **N=4** | **N=5** | N=2 | N=5 | N=6 | **N=9** | **N=15** | **N=11** |

The percentage of the subjects experiencing an improvement in skin hydration, seborrhea reduction and good skin condition maintenance was 82%, 82% and 90%, respectively.

The average improvement in the group declaring an improvement was 40% (increase in hydration), 29% (seborrhea reduction) and 39% (good skin condition). Table 4 shows data concerning the subjects' subjective assessment of an improvement in skin hydration, seborrhea reduction and good skin condition maintenance.

**Table 4. Data concerning the subjects' subjective assessment of an improvement in skin hydration, seborrhea reduction and good skin condition maintenance.**

| | Skin hydration | | | Seborrhea reduction | | | Good skin condition | | |
|---|---|---|---|---|---|---|---|---|---|
| | forehead | chin | cheeks | **forehead** | **chin** | **cheeks** | forehead | chin | cheeks |
| **Average improvement in the group declaring an improvement (size of the group)** | 37.5% (N=23) | 38.0% (N=20) | 43.2% (N=23) | **32.5% (N=23)** | **24.1 % (N=18)** | **29.6% (N=20)** | 39.7% (N=23) | 33.9% (N=24) | 43.8% (N=23) |
| Size of the group with no changes | N=2 | N=5 | N=1 | **N=2** | **N=7** | **N=3** | N=1 | N=2 | N=2 |
| **Average deterioration in the group declaring deterioration (size of the group)** | 20.0% (N=2) | 15.0% (N=2) | 36.7% (N=3) | **0.0% (N=0)** | **0.0% (N=0)** | **10.0% (N=1)** | 22.5% (N=2) | 0.0% (N=0) | 20.0% (N=1) |
| Size of the group with no symptoms | N=0 | N=0 | N=0 | **N=2** | **N=2** | **N=3** | N=1 | N=1 | N=1 |

### Summary and conclusions

Applying the cosmetic composition according to the invention by persons having acne skin resulted in an objective improvement in hydration and TEWL and seborrhea reduction in those persons. An improvement in this respect is extremely desirable for acne skin.

In the subjects' opinion, the tested cosmetic composition reduced acne lesions (blackheads, papules, pustules and erythema) in about 3/4 of the subjects at the level of about 40%. Additionally, it improved hydration by 40%, reduced seborrhea by 29% in 82% of the subjects and maintained the skin in good condition in 90% of the subjects.

It results from the study that the cosmetic composition according to the invention is suitable for the care of acne skin because it maintains the skin in good condition by reducing sebum secretion and ensuring proper hydration of the skin. This also translates into a decrease in the number of skin lesions, i.e. blackheads, papules and pustules, typical of acne-affected skin.

The cosmetic composition according to the invention is a suitable preparation supplementing the care of acne skin during dermatological therapies, e.g. by retinoids.

The cosmetic composition according to the invention may further have an anti-inflammatory, antibacterial, healing-accelerating, irritation- and redness-relieving, coating (occlusive) effects on pathologically changed skin, thus supporting the treatment of atopic dermatitis, psoriasis, acne rosacea and common acne, burn wounds, sunburn, skin ulcers, etc.

The solutions according to the invention can be used in the cosmetic and pharmaceutical industries, especially in the development of cosmetic products containing raw materials derived from natural sources, which are currently highly desired by consumers.

The cosmetic composition according to the invention will enjoy great interest from companies in the cosmetic and pharmaceutical industries due to its multidirectional conditioning effect on both healthy skin and skin with lesions.

## Claims

1. A cosmetic composition comprising:
- 0.1% to 50% by weight of ovine colostrum being the active substance; and
- a cosmetic base comprising at least one preservative, at least one emulsifier and at least one solvent.

2. The composition according to claim 1, wherein the composition comprises the at least one preservative in a total amount of 0.001% to 2.5% by weight relative to the total mass of the composition.

3. The composition according to claim 1 or claim 2, wherein the composition comprises the at least one emulsifier in a total amount of 1% to 25% by weight relative to the total mass of the composition.

4. The composition according to any one of claims 1 to 3, wherein the composition comprises the at least one solvent in an amount that is a complement to 100% by weight relative to the total mass of the composition.

5. The composition according to any one of claims 1 to 4, wherein the cosmetic base additionally comprises at least one ingredient selected from the group consisting of:
- at least one thickener, preferably in a total amount of 1% to 60% by weight,
- at least one humectant, preferably in a total amount of 1% to 50% by weight,
- at least one emollient, preferably in a total amount of 1% to 25% by weight,
- at least one antioxidant, preferably in a total amount of 0.01% to 10% by weight,
- at least one fragrance, preferably in a total amount of 0.01% to 3% by weight,
- at least one pH regulator, preferably in a total amount sufficient to obtain a pH value in the range of 4.5 to 6.0,
- at least one surfactant, preferably selected from the group consisting of anionic, cationic, amphoteric and non-ionic surfactants in a total amount of 0.1% to 5% by weight,
- at least one ultraviolet filter, preferably in a total amount of 1% to 30% by weight,
- at least one silicone, preferably in a total amount of 1% to 50% by weight,
- at least one extender, preferably in a total amount of 0.1% to 10% by weight, and
- at least one colourant, preferably in a total amount of 0.1% to 5% by weight,
wherein all amounts are given relative to the total mass of the composition.

6. The composition according to any one of claims 1 to 5, wherein ovine colostrum is in the form of lyophilizate, in liquid form or in partially concentrated form.

7. The composition according to any one of claims 1 to 6, wherein the composition is in the form of: cream, ointment, serum, facial and body mask, exfoliator, balm, gel, shampoo, hair mask, hair conditioner, lip care stick or nail conditioner.

8. A method of producing the composition defined in any one of claims 1 to 7, wherein the method comprises the following steps:
a) introducing a solvent forming phase I into a mixer and heating it to a temperature falling within the range of 20 to 80°C;
b) introducing an emulsifier forming phase II into a melting crucible and then heating it to a temperature falling within the range of 20 to 80°C and mixing to obtain a homogenous phase;
c) dosing phase II from step b) to phase I from step a) and simultaneously mixing to obtain phase III in a homogenous form;
d) adding, to phase III obtained in step c), successively: ovine colostrum and a preservative to obtain the composition, wherein each time after adding a successive ingredient, phase III is thoroughly mixed until it is homogenous.

9. The method of producing the composition according to claim 8, wherein the method further comprises, after step d), the step of:
e) cooling down the composition obtained in step d) to room temperature.

10. The method of producing the composition according to claim 8 or 9, wherein the method further comprises after step d) or e), the step of:
f) adding a pH regulator in an amount sufficient to obtain a pH value in the range of 4.5 to 6.0 to the composition obtained in step d) or e), wherein the adding is carried out in portions and with mixing.

11. The method of producing the composition according to any one of claims 8 to 10, wherein the method further comprises, between steps c) and d), the step of:
c') cooling down phase III obtained in step c) to a temperature below 40°C.

12. The method of producing the composition according to any one of claims 8 to 11, wherein in step a), at least one additional ingredient forming phase I is introduced into the mixer while mixing, which is selected from the group consisting of:
- at least one thickener, preferably in a total amount of 1% to 60% by weight,
- at least one humectant, preferably in a total amount of 1% to 50% by weight, and
- at least one surfactant, preferably in a total amount of 0.1% to 5% by weight,
wherein all amounts are given relative to the total mass of the composition.

13. The method of producing the composition according to any one of claims 8 to 12, wherein in step b), at least one additional ingredient forming phase II is introduced into the melting crucible, which is selected from the group consisting of:
- at least one antioxidant, preferably in a total amount of 0.01% to 10% by weight relative to the total mass of the composition, and
- at least one emollient, preferably in a total amount of 1% to 25% by weight relative to the total mass of the composition.

14. The method of producing the composition according to any one of claims 8 to 13, wherein in step d), at least one additional ingredient is added, which is selected from the group consisting of:
- at least one fragrance, preferably in a total amount of 0.01% to 3% by weight,
- at least one colourant, preferably in a total amount of 0.1% to 5% by weight,
- at least one antioxidant, wherein preferably the total amount of the at least one antioxidant added in step b) and/or in step d) is 0.01% to 10% by weight,
- at least one ultraviolet filter, preferably in a total amount of 1% to 30% by weight,
- at least one silicone, preferably in a total amount of 1% to 50% by weight,
- at least one extender, preferably in a total amount of 0.1% to 10% by weight, and
- at least one humectant, wherein preferably the total amount of the at least one humectant added in step a) and/or step d) is 1% to 50% by weight,
wherein all amounts are given relative to the total mass of the composition.

15. The method of producing the composition according to any one of claims 8 to 14, wherein the at least one emulsifier is introduced in step b) in such an amount that the total amount of the at least one emulsifier in the composition is 1% to 25% by weight relative to the total mass of the composition.

16. The method of producing the composition according to any one of claims 8 to 15, wherein ovine colostrum is added in step d) in such an amount that its amount in the composition is 0.1% to 50% by weight relative to the total mass of the composition.

17. The method of producing the composition according to any one of claims 8 to 16, wherein the at least one preservative is added in step d) in such an amount that the total amount of the at least one preservative in the composition is 0.001% to 2.5% by weight relative to the total mass of the composition.

18. The method of producing the composition according to any one of claims 8 to 17, wherein the at least one solvent is introduced in step a) in such an amount that the total amount of the at least one solvent in the composition is a complement to 100% by weight relative to the total mass of the composition.

19. A use of the composition defined in any one of claims 1 to 7, for improving skin condition, wherein improving skin condition comprises: increasing skin hydration, increasing skin elasticity, reducing redness, regenerating aging skin, reducing skin hypersensitivity, evening out skin tone and smoothing skin pores.

20. The composition defined in any one of claims 1 to 7, for use in the topical treatment of acne.
